# EUROPEAN PATENT APPLICATION

(11) **EP 2 410 336 A1**
(43) Date of publication of application: **25.01.2012**
(21) Application number: 09841942.7
(22) Date of filing: 15.04.2009
(51) Int. Cl.: G01N 33/574

(54) **DIAGNOSTIC MARKER FOR BREAST CANCER, HAVING THIOREDOXIN-1 AS AN ACTIVE INGREDIENT, AND DIAGNOSTIC KIT FOR BREAST CANCER USING SAME**

(30) Priority: 16.03.2009 KR 20090022291
(71) Applicant: PAICHAI UNIVERSITY INDUSTRY-ACADEMIC COOPERATION FOUNDATION, Seo-Gu, Daejeon 302-735 (KR)
(72) Inventor: KIM, Il Han, Daejeon 305-773 (KR)
(74) Representative: Schwabe - Sandmair - Marx
(86) International application number: PCT/KR2009/001944
(87) International publication number: WO 2010/107158

(57) **Abstract**

The present invention relates to a diagnostic marker for breast cancer, having thioredoxin-1 as an active ingredient, and a diagnostic kit for breast cancer using same. The thioredoxin-1 according to the present invention is overexpressed in human breast cancer tissue so as to enable the early diagnosis of breast cancer or the early prediction prognosis of breast cancer, and therefore has a valuable use as a diagnostic marker for breast cancer.

## Description

### [Technical Field]

The present invention relates to a diagnostic marker for breast cancer, comprising thioredoxin-1 as an active ingredient, and a diagnostic kit for breast cancer using the same.

### [Background Art]

Various etiologic factors for breast cancer are now being mentioned, including female hormones, cancer history, family history, lack of having borne a child, dietary habit, etc., but no clear relationship with these has been proven thus far. Recently, breast cancer cases in South Korea are reported to have rapidly increased. A survey report of the Statistics Korea has it that breast cancer cases exceeded uterine cervical cancer cases in 1998 and accounted for 16.1% of women's cancer cases in 2001, ranking first before stomach cancer. In addition, the incident rate of breast cancer was 11.1% between 2001 and 2002, which was greater than that of other cancers. Now, the high incident rate of breast cancer is thought to be associated with various factors. Among them are a low birthrate, breastfeeding for a short period of time, early menarche, late menopause, the westernization of dietary habits, and the pollution of living environment. Further, glandular tissue of women who had been stimulated by female hormones at rapidly increased frequency at the time of their physiologically active change was increased in sensitivity and these individuals may be confronted with a greater likelihood of being struck by breast cancer.

The incidence of breast cancer and the mortality from breast cancer in Korea are expected to increase for a significant period of time in the future in consideration of the trend of because of the westernization of living. As they grow, breast cancer cells, like other cancer cells, generally infiltrate adjacent tissues or metastasize into the lymph nodes. In most breast cancer cases, the patients have had few detectable symptoms nor do they examine their breasts by themselves. Therefore, it is very important to effectively diagnose early breast cancer to reduce mortality from breast cancer.

For the diagnosis of breast cancer, various methods are used in combination. So far, breast self-examination has accounted for 70% of breast cancer cases. However, it is impossible to discriminate malignant breast tumors from benign tumors by giving a breast self-examination. A number of breast cancer screening tests have been employed including X-ray mammography, ultrasound, fine-needle aspiration cytology, and magnetic resonance imaging. Eventually, biopsy is employed to diagnose breast cancer. Mammographic screening for breast cancer uses X-rays to examine the breast. Mammography is effective at determining whether breast tumors are benign or malignant and can detect hidden tumors. This method is one of the most effective techniques by which early breast cancer can be diagnosed before the detection of a lump by breast self-examination. However, mammography is disadvantageous in that diagnostic accuracy is lowered for a breast in which the mammary gland is highly developed or which is a small and dense breast with highly fibrous tissue, like the breasts of young women. In addition, there is a dispute waging about thatmore frequent mammograms run the risk of significantly increasing breast cancer because of the radiation. As an alternative to mammography, ultrasonography is employed. Ultrasonography can effectively discriminate cystis from lumps, but is ineffective in telling malignant tumors from benign tumors.

To supplement such breast screening methods, attempts have been made to use blood tumor marker levels to diagnose breast cancer. Although studied for their values as diagnosis or prognosis factors, the application of conventional tumor markers is accompanied by limitations, and there are no officially recommended breast cancer markers.

Thioredoxin (Trx) is a 12 kDa oxidoreductase thatis kept in the reduced state by thioredoxin reductase in a NADPH-dependent reaction. There are two kinds of mammal thioredoxins: thioredoxin 1 (Trx1) and thioredoxin 2 (Trx2). Thioredoxins act as electron donors to peroxidases so that, for example, toxic hydrogen peroxide can be eliminated. Also, thioredoxins provide electrons for ribonucleotide reductase. Thioredoxins plays a role in binding transcription elements to DNA in bacteria while they have an influence on the activity of NF-kB (nuclear transcription factor kB) in eukaryotes. Therefore, thioredoxins are associated with cell death and tumor cells, and play an important role in regulating the growth of tumor cells. Serving as a general disulfide oxidoreductase, thioredoxin facilitates the reduction of other proteins by a redox mechanism based on reversible reduction of a disulfide to two cysteine thiol groups, thereby recovering the normal function of the proteins. In mammalian cells, thioredoxin 1 and 2 are also involved in the regulation of nitric oxide levels and thus in cell death. Therefore, the enzymes are potentially important in conjunction with the onset of many diseases including inflammatory diseases, heart failure, cancer, etc. Immunohistochemical analysis with anti-thioredoxin antibodies revealed the expression of thioredoxins in cancer cells in various tissues such as the liver, colon, pancreas, and the uterine cervix, indicating the implication of thioredoxins in oncogenesis.

There has been a need for a diagnostic marker for breast cancer that allows the accurate diagnosis of early breast cancer and allows the prognosis of breast cancer to be made. Almost few studies have been done on the use of thioredoxin 1 as a diagnostic marker for breast cancer.

### [Disclosure]

### [Technical Problem]

Leading to the present invention, intensive and thorough research into the diagnosis and prognosis of breast cancer conducted by the present inventors, resulted in the finding that thioredoxin 1 is expressed at a low level in normal breast tissues, but at a high level in breast cancer tissues.

### [Technical Solution]

The present invention provides a diagnostic marker for breast cancer, comprising thioredoxin 1 as an active ingredient, and a diagnostic kit for breast cancer, using the same.

### [Description of Drawings]

FIG. 1 is a graph showing expression profiles of thioredoxin 1 transcript in 48 different human normal tissues selected from individuals of different ethnicity as measured by 96-well HMRT qPCR assay.
FIG. 2 is a graph showing mRNA levels of thioredoxin 1 and 2 in eight solid cancers (breast, colon, kidney, liver, lung, ovary, prostate, and thyroid) as measured by CSRT 96-I arrays.
FIG. 3 is of graphs showing mRNA levels of thioredoxin 1 and 2 in breast cancer (A) and levels of induction fold obtained therefrom (B).
FIG. 4 shows the induction fold of mRNA expression of thioredoxin 1 in human breast cancer as a scatter dot plot (A), the correlation between the mRNA expression of thioredoxin 1 and the progression of cancer as a box-and-whisker plot (B), and the induction fold of mRNA expression of thioredoxin in subdivided cancer (stage I, IIA, IIB, IIIA, IIIB, IIIC, and IV) as a plot (C).
FIG. 5 shows the expression level of thioredoxin 1 in human breast cancerous tissues as measured by Western blot assay.
FIG. 6 shows the expression levels of thioredoxin 1 in four normal tissues and four cancerous tissues from different individuals, as measured by Western blotting assay.
FIG. 7 shows the expression levels of thioredoxin 1 in paired sets of breast tissue (paired normal and primary cancer from the same individual; paired primary and metastatic cancer from the same individual) and paired sets of other cancerous tissues (lung and colon), as measured by Western blotting assay.

### [Best Mode]

The present invention provides a diagnostic marker for breast cancer, comprising thioredoxin 1 as an active ingredient.

Also, the present invention provides a diagnostic kit for breast cancer, comprising an antibody against thioredoxin 1.

Further, the present invention provides a method for detecting thioredoxin 1 in breast tissues using an antibody to thioredoxin 1.

Below, a detailed description will be given of the present invention.

In the present invention, the expression levels of thioredoxin 1 in human normal tissue and cancerous tissue were examined. Thioredoxin 1 was found to be expressed at the lowest level in normal breast tissue among 48 different normal human tissues, and at a higher level in breast cancer tissue than in other cancerous tissue, as measured by qRT-PCR and Western blotting. In addition, the more progressed the cancer, the higher the induction fold of mRNA expression of thioredoxin 1. Thus, the induction fold of mRNA expression of thioredoxin 1 becomes high in stage II∼IV breast cancer, particularly stage IV breast cancer, that is, metastatic breast cancer. Further, the induction fold of mRNA expression of thioredoxin 1 is closely associated with the malignancy of cancer as it increases with the progression of cancer. Therefore, the induction fold of mRNA expression of Thoredoxin 1 is associated with subdivision of cancer stages.

Found to be overexpressed in human breast cancer tissues, as described above, thioredoxin 1 allows the diagnosis and prognosis of breast cancer and thus is useful as a diagnostic marker for breast cancer.

The diagnostic kit for breast cancer, comprising an antibody against thioredoxin 1 in accordance with the present invention may be readily prepared using the marker.

The diagnostic kit for breast cancer of the present invention may comprise an antibody specifically binding to thioredoxin 1, a secondary antibody conjugate with a label that can react with a substrate to cause a chromatic change, a substrate solution which develops a color upon reaction with the label, a washing buffer and a reaction stop buffer.

The label conjugated to the secondary antibody is preferably a coloring agent which can bring about a color change as it reacts with its substrate. Representative among them are HRP (horseradish peroxidase), alkaline phosphatase, colloid gold, fluorescein such as FITC (poly L-lysine-fluorescein isothiocyanate) and RITC (rhodamine-B-isothiocyanate), and dye.

As for the substrate solution, it is dependent on the label. Examples include TMB (3,3',5,5'-tetramethyl bezidine), ABTS [2,2'-azino-bis(3-ethylbenzothiazoline-6-sulfonic acid)], and OPD (o-phenylenediamine). The coloring substrate is preferably provided in the form of a solution in buffer (0.1M NaOAc, pH 5.5).

Preferably, phosphate buffer, NaCl and Tween 20 are contained in the washing solution. More preferable is a solution (POST) containing 0.02M phosphate buffer, 0.13M NaCl, and 0.05% Tween 20. After the antibody is allowed to react with the antigen, the antigen-antibody complex is treated with the secondary antibody conjugate, followed by immobilization and then washing 3∼6 times with the washing solution. A sulfuric acid solution may be used to stop the enzymatic reaction.

In accordance with one embodiment of the present invention, the diagnosis or prognosis of breast cancer can be readily predicted with high accuracy by detecting the breast cancer marker with an antigen-antibody reaction using an antibody specifically binding to thioredoxin 1. For example, a protein preparation containing thioredoxin 1 is separated on SDS-PAGE, and transferred and fixed onto an immobilizer which is then treated with an antibody against thioredoxin 1 to determine the expression level of thioredoxin 1. That is, when the expression level of thioredoxin 1 in the breast tissue of interest is measured, the breast tissue is diagnosed with cancer or predicted to become cancerous when the thioredoxin 1 expression level is higher than that of normal breast tissue.

Hither expression levels of thioredoxin 1 are detected in stage II∼IV breast cancer tissues than in stage I breast cancer or normal breast tissues.

Examples of the immobilizer include a nitrocellulose membrane, a PVDF (polyvinylidene difluoride) membrane, a 96-well plate formed of polyvinyl resin or polystyrene resin, and a slide glass.

The antigen-antibody binding reaction may be assayed using a typical method such as ELISA, radioimmnoassay (RIA), sandwich assay, Western blotting, immunoprecipitation immnohistochemical staining, immunofluorescence assay, enzyme-substrate coloring assay, and antigen-antibody aggregation.

### [Mode for Invention]

A better understanding of the present invention may be obtained through the following examples which are set forth to illustrate, but are not to be construed as limiting the present invention.

### EXAMPLE 1 :qRT-PCR Assay

For expression profiling of human target genes, qRT-PCR (Quantitative real-time Polymerase Chain Reaction) arrays were used to conduct the following reactions.

Human Major 48 tissues real-time (HMRT) qPCR arrays, Cancer Survey real-time (CSRT 96-I) qPCR arrays, and Human Breast Cancer real-time (BCRT I-V) qPCR arrays, all purchased from OriGene (OriGene Technologies, Inc, Rockville, MD, USA) were used. Simultaneous examination of the expression of target genes in 48 different tissues was performed using the HMRT array, which consisted of panels of first-strand cDNA from human tissues selected from individuals of different ethnicity. Expression levels of target genes in eight different cancers (breast, colon, kidney, liver, lung, ovary, prostate, and thyroid) were measured using the CSRT array consisting of 12 samples from each cancer type with cancer stages varying from I to IV. Expression of target genes in breast cancer was examined using four different sets of arrays (BCRT I-IV) for 192 test samples and using the CSRT 96-1 array for 12 test samples. In the 204 samples, cancer grading was distributed as follows: 19 as stage 0 (normal); 37 as stage I; 76 as stage II; 60 as stage III; and 12 as stage IV. The cancer tissue types consisted of ductal (*n* = 154), lobular (n = 13), metastatic (n = 12), and other histological types of cancer (n = 25), including medullary, mucinous, tubular, recurrent, and papillary. More clinicopathological information for each patient is described in OriGene's product sheet.

PCR was performed in 96-well optical plates using iCycler (Bio-Rad Laboratories, Hercules, CA, USA) with primers specific for Trx1, Trx2, β-actin, GAPDH (glyceraldehyde 3-phosphate dehydrogenase), and iQ SYBR Green Supermix (Bio-Rad). The resulting fluorescence proportional to the amount of amplified DNA was measured at the end of each elongation phase at 530 nm. A standard graph of C_{T} (the point at which the fluorescence crosses the threshold) values obtained from serially diluted target genes was constructed for all reactions to ensure that they were amplified and reported in proportion to the template. C_{T} values were converted to gene copy number of the template cDNA using the equation 2^{ΔΔCT}. The ΔC_{T} is the abundance of cDNAs for transcripts of each gene normalized to the β-actin and GAPDH at each time point. The ΔΔC_{T} is obtained by subtracting a calibrator value for each gene transcript being assayed. In parallel with each cDNA sample, standard curves were constructed to correlate C_{T} values using serial dilutions of the target gene. The property of the standard curve was evaluated from the slope and the correlation coefficient. Quantification was performed by comparing the fluorescence of a PCR product of unknown concentration with the fluorescence of several dilutions. Melting curve analysis was used for product validation. The primers for β-actin and GAPDH were purchased from Origene. Other primer sequences are summarized in Table 1.

**TABLE 1**

| Sequence of Primers for Real-Time PCR Amplification | Direction | Primer Sequence (5'→3') |
|---|---|---|
| Human thioredoxin 1 (Trx 1) | Forward | ctgcttttcaggaagccttg |
| | Reverse | tgttggcatgcatttgactt |
| Human thioredoxin 2(Trx 2) | Forward | agcccggacaatatacacca |
| | Reverse | aatatccaccttggccatca |

### 1-1. Transcript Level of Thioredoxin 1 in 48 Different Normal Human Tissues

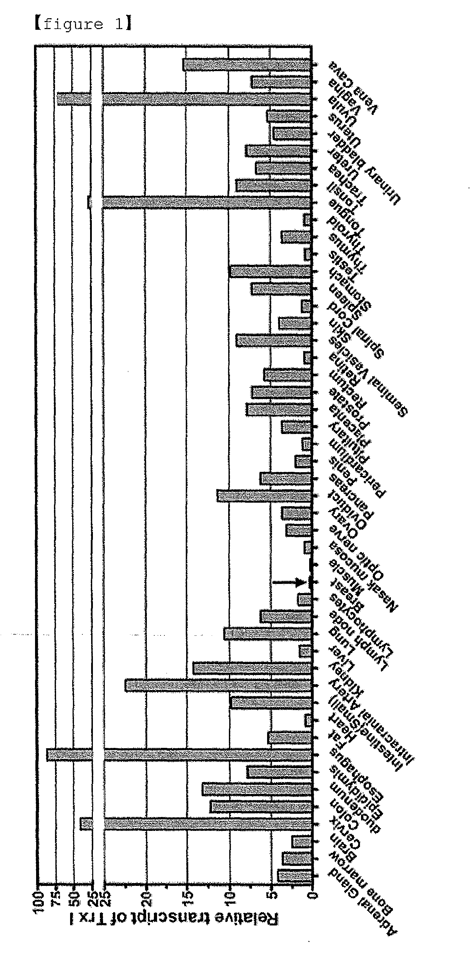

Transcript levels of thioredoxin 1 in 48 different human normal tissues selected from individuals of different ethnicities were determined using 96-well HMRT qPCR arrays, and the results are given in FIG. 1.

As can be seen in FIG. 1, thioredoxin 1 was expressed at the lowest level (0.24 × 10⁻⁴ pg) in breast tissue among the 48 major human tissues.

### 1-2. Levels of mRNA of Thioredoxin 1 and 2 in Human Solid Cancerous Tissues

The expression profiles of thioredoxin 1 and 2 in eight solid cancers (breast, colon, kidney, liver, lung, ovary, prostate, and thyroid) were made using the CSRT 96-1 array.

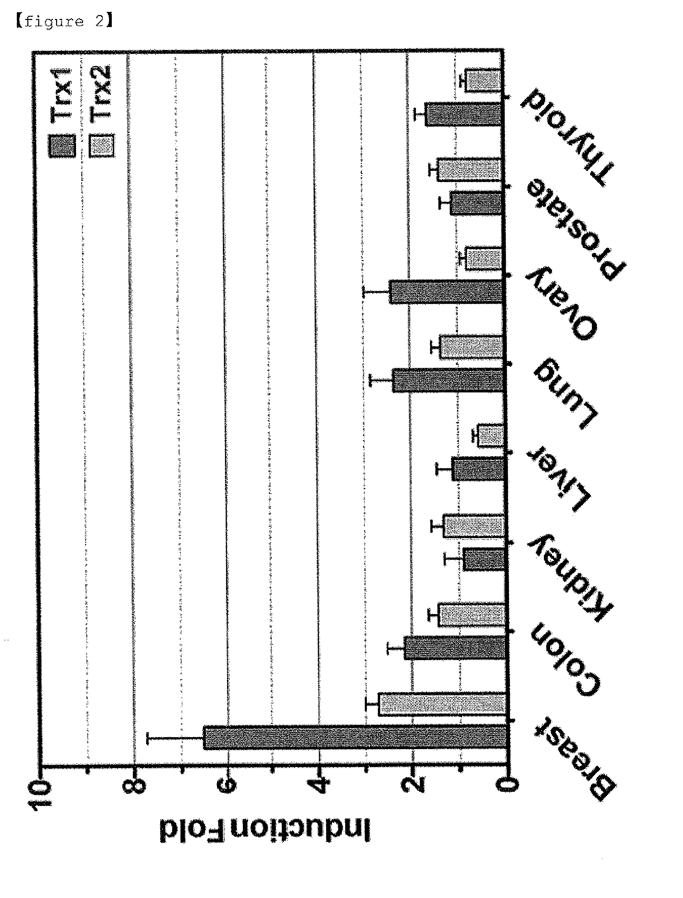

The results are given in FIG. 2.

As shown in FIG. 2, thioredoxin 1 was expressed at the highest level in breast cancer (6.47 ± 1.22) among the eight solid cancers, whereas thioredoxin 2 was not preferentially expressed in breast cancer (2.72 ± 0.28) (P = 0.0067).

### 1-3. Levels of mRNA of Thioredoxin 1 and 2 in Human Breast Cankerous Tissues

To examine the expression profile of thioredoxin 1 in breast cancer, the mRNA levels of thioredoxin 1 and 2 in breast cancer were quantified using a 48-well BCRT II array. The induction fold was obtained from the mRNA concentrations of thioredoxin 1 and 2.

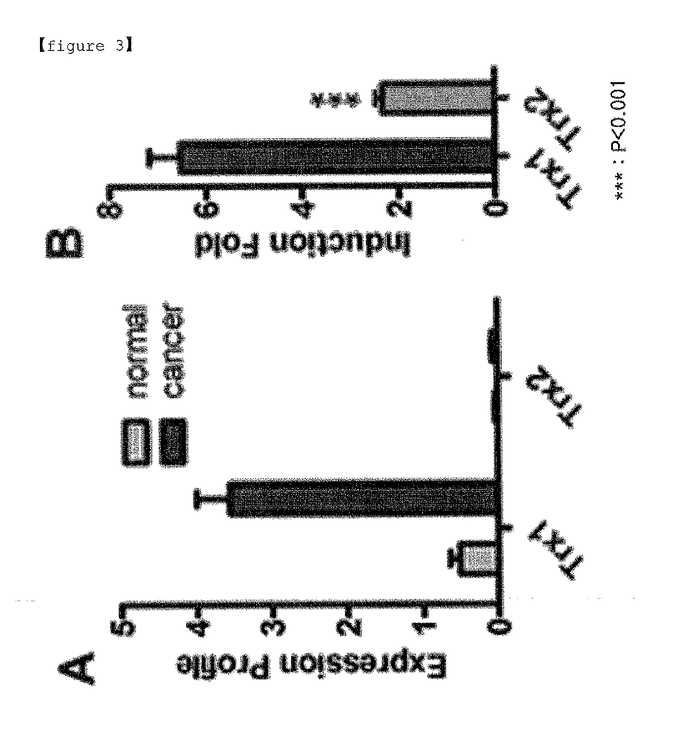

The mRNA concentrations (A) of thioredoxin 1 and 2 in breast cancer and the induction folds obtained therefrom are shown in FIG. 3.

As can be seen in FIG. 3, the mRNA levels of thioredoxin 1 were much higher than those of thioredoxin 2 in both normal breast and breast cancer tissues. Also, the higher-induction fold of thioredoxin in malignant tissue is depicted compared with thioredoxin 2.

### 1-4. Correlation between Grade of Human Breast Cancer and Thioredoxin 1

To evaluate the correlation of thioredoxin with the progression of breast cancer, mRNA levels in 204 samples of normal and malignant breast tissues ranging from 0 to IV grade were assessed using five different sets of qRT-PCR arrays [Cancer Survey real-time (CSRT 96-I) qPCR array (n=9), and human breast cancer real-time (BCRT I-V) qPCR array I∼V (n=176)], the induction fold from normal (grade 0) to malignant (grade I, II, III, IV) was determined.

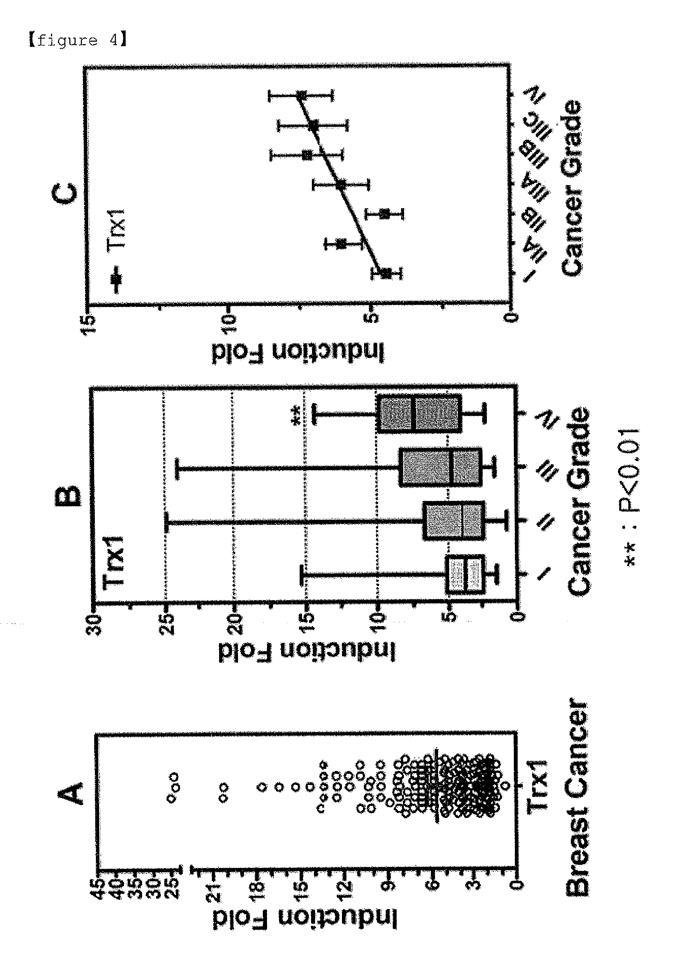

In FIG. 4, Induction fold data for mRNA expression of thioredoxin 1 in human breast cancer were displayed as a scatter dot plot (A), the correlation of mRNA expression of thioredoxin with cancer grade was represented as box-and-whisker plots (B), and data for the induction fold of mRNA expression of thioredoxin 1 in subdivided human breast cancer (stage I, IIA, IIB, IIIA, IIIB, IIIC, and IV) was plotted (C).

In breast cancer, as can be seen in FIG. 4, 2-fold overexpression of thioredoxin 1 occurred in 168 of 185 cases (90.8%). Mean ± SEM induction fold was 5.64 ± 0.33 for thioredoxin 1 (A). There was a significant correlation between the induction fold of thioredoxin 1 and increasing cancer grade. The higher the grade of cancer, the higher the induction fold of mRNA expression of thioredoxin 1. The induction fold was closely correlated with stage II∼IV breast cancer, especially stage IV cancer, that is, metastatic cancer (B). Further, induction fold was associated with subdivisions of cancer stages (P = 0.0191) (C).

### EXAMPLE 2 : Western Blotting Analysis

To examine the expression level of thioredoxin 1 in human breast cancer tissues with Western blotting analysis, the following experiment was conducted.

Total membrane and soluble proteins from clinically defined human cancer and normal tissues were obtained from Capital Biosciences (Gaithersburg, MD, USA). Proteins from different individuals and matched paired individuals (normal tissue and primary cancers; primary and metastatic cancers) were used for immunological analysis. The clinical and pathological traits of the cancers are summarized in Table 2, below.

The total membrane and soluble protein lysates (15 µg) from seven cancer tissue types (brain, breast, colon, kidney, liver, lung, and ovary) were loaded into reducing and non-reducing SDS-PAGE, followed by Western blot analysis using an Amersham ECL Western blotting system (GE Healthcare, Chalfont St. Giles, United Kingdom). Anti-thioredoxin 1, and anti-copper/zinc (Cu/Zn) superoxide dismutase (SOD) rabbit polyclonal antibodies that have cross-reactivity with the corresponding human protein were purchased from AbFrontier (Seoul, Korea). Samples were fractionated by electrophoresis on a 4% to 20% gradient SDS-PAGE (GenScript Corp., Piscataway, NJ, USA) and transferred onto PVDF (polyvinylidene difluoride) membranes (Millipore, Billerica, MA, USA). The membranes were blocked and incubated at room temperature for 2 hours with an antibody (1:1000 by volume) in PBS containing 0.1% Tween 20. After washing many times, the membranes were incubated with horseradish peroxidise-conjugated polyclonal goat anti-rabbit IgG antibody (1:2000 by volume). Then, the membranes were washed in PBS, and the chemiluminescent substrate was added. The membranes were stained with Coomassie Blue R-250 for verification of the loading sample.

**TABLE 2**

| Sample | Tissue | Appearance | Age/Gender | Disgnosis |
|---|---|---|---|---|
| BRN0 | Brain | Normal | 26/M | Normal |
| BRC0 | Brain | Tumor | 40/M | Astrocytoma |
| BENO-4 | Breast | Normal | 82/F.45/F. 56/F.64/F. 76/F | Normal |
| BEC0 | Breast | Tumor | 47/F | Medullary Carcinoma, Well Differentiated |
| BEC1 | Breast | Tumor | 40/F | Invasive Lobular Carcinoma |
| BEC2 | Breast | Tumor | 42/F | Adenocarcinoma, Moderately Differentiated |
| BEC3 | Breast | Tumor | 42/F | Fibroadenoma |
| BEC4 | Breast | Tumor | | 50/F Infiltrative Ductal Carcinoma |
| CLN0 | Colon | Normal | 60/F | Normal |
| CLCO | Colon | Tumor | 48/M | Adenocarcinoma, Well Differentiated |
| KDN0 | Kidney | Normal | 83/F | Normal |
| KDCO | Kidney | Tumor | 43/F | Granular Cell Carcinoma |
| LVN0 | Liver | Normal | 30/M | Normal |
| LVC0 | Liver | Tumor | 65/M | Hepatic Cellular Carcinoma |
| LUN0-4 | Lung | Normal | 24/F.26/M. 66/M.71/M. 76/F | Normal |
| LUC0 | Lung | Tumor | 72/M | Squamous Cell Carcinoma |
| LUC1 | Lung | Tumor | 33/M | Squamous Cell Carcinoma, Moderately Differenciated |
| LUC2 | Lung | Tumor | 51/F | Adenocarcinoma, Moderately Differentiated |
| LUC3 | Lung | Tumor | 58/M | Squamous Cell Carcinoma, Moderately Differenciated |
| LUC4 | Lung | Tumor | 61/M | Adenocarcinoma |
| OVN0-4 | Ovary | Normal | 74/F.37/F. 62/F.69/F. N/A/F | Normal |
| OVC0 | Ovary | Tumor | 51/F | Cystoadenocarcinoma |
| OVC1 | Ovary | Tumor | 42/F | Granular Cell Carcinoma |
| OVC2 | Ovary | Tumor | 51/F | Cystoadenoma |
| OVC3 | Ovary | Tumor | 57/F | Leiomyosarcoma, Well Differentiated |
| OVC4 | Ovary | Tumor | Adult/F | Clear Cell Adenocarcinoma |
| BE1N | Breast | Adjacent Normal | 70/F, patient | same Normal |
| BE1P | Breast | Primary Tumor | | Invasive Ductal Carcinoma |
| BE2P | Breast | Primary Tumor | 59/F, same patient | Breast Carcinoma |
| BE2M | Breast | Metastatic Tumor | | Breast Tumor Metastasized to Lung |
| CL1N | Colon | Adjacent Normal | 62/F, same patient | Normal |
| CL1P | Colon | Primary Tumor | | Adenocarcinoma |
| CL2P | Colon | Primary Tumor | 66/F, same patient | Adenocarcinoma |
| CL2M | Colon | Metastatic Tumor | | Colon Tumor Metastasized to Lymph Node |
| LU1N | Lung | Adjacent Normal | 46/M, same patient | Normal |
| LU1P | Lung | Primary Tumor | | Squamous Cell Carcinoma |
| LU2P | Lung | Primary Tumor | 75/M, same patient | Squamous Cell Carcinoma |
| LU2M | Lung | Metastatic Tumor | | Lung Tumor Metastasized to Lymph Node |

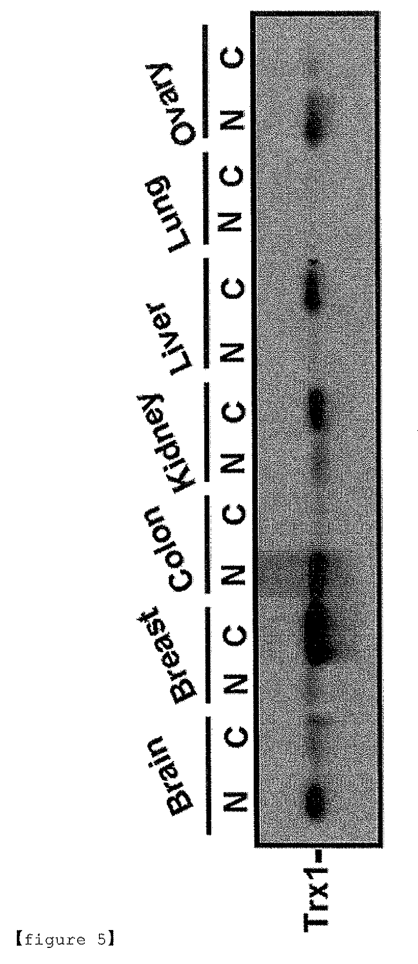

The results are shown in FIG. 5.

As shown in FIG. 5, thioredoxin 1 was expressed at the highest level in human breast cancerous tissues.

### 2-1. Expression Levels of Thioredoxin 1 in Human Normal Tissues and Cancerous Tissues (Breast, Lung, Ovary)

The expression levels of thioredoxin 1 in four normal tissues and four cancerous tissues from different individuals were analyzed by Western blotting.

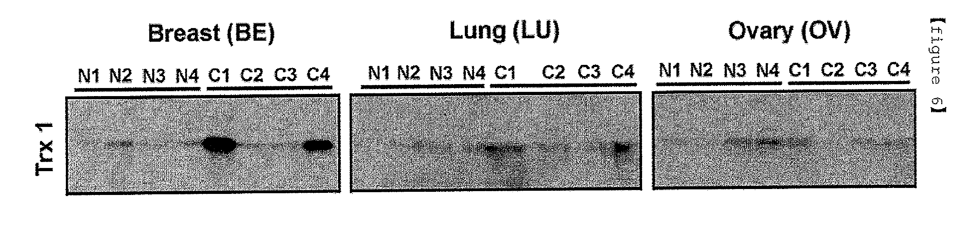

The results are given in FIG. 6.

As can be seen in FIG. 6, thioredoxin 1 was overexpressed in breast cancer.

### 2-2. Expression Levels of Thioredoxin 1 in Paired Sets of Breast Tissue and Paired Sets of Other Tissues (Lung and Colon)

The expression levels of thioredoxin 1 in the paired sets of breast tissue (paired normal and primary cancer from the same individual; paired primary and metastatic cancer from the same individual) and the paired sets of other tissues (lung and colon) were analyzed by Western blotting. Cu/Zn SOD is used as positive coltrol.

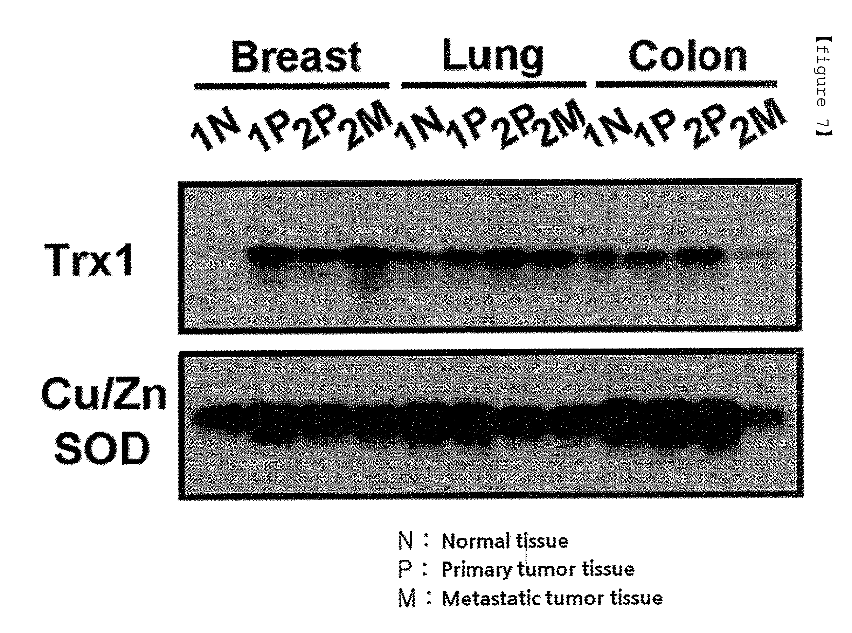

The results are shown in FIG. 7.

As can be seen in FIG. 7, thioredoxin 1 was preferentially overexpressed in breast cancer tissues over the other tissues.

### [Industrial Applicability]

Being overexpressed in human breast cancerous tissues, thioredoxin 1 can be used as a diagnostic marker which allows the accurate diagnosis and prognosis of breast cancer to be made.

## Claims

1. A diagnostic marker for breast cancer, comprising thioredoxin 1 as an active ingredient.

2. The diagnostic marker of claim 1, wherein the marker is specific for stage II∼IV breast cancer.

3. A diagnostic kit for breast cancer, comprising an antibody specifically binding to thioredoxin 1.

4. A method for detecting thioredoxin 1 in breast tissues, comprising an antigen-antibody reaction using an antibody specifically binding to thioredoxin 1

5. The method of claim 4, wherein thioredoxin 1 is expressed at a higher level in stage II∼IV breast cancerous tissues than in stage I breast cancerous tissues or normal breast tissues

6. A method for diagnosis or prognosis of breast cancer using the marker of claim 1, comprising:
1) measuring an expression level of thioredoxin 1 in a breast tissue of interest; and
2) comparing the measured expression level of thioredoxin 1 in the breast tissue of interest with that in a normal breast tissue to diagnose the breast tissue of interest with caner or to predict a likelihood of becoming cancerous when the measured expression level is higher than that of normal breast tissue.

7. The method of claim 6, wherein the expression level of thioredoxin 1 is higher in stage II∼IV breast cancerous tissues than in stage I breast cancerous tissues or normal breast tissues.
